# EUROPÄISCHE PATENTSCHRIFT

(11) **EP 0 315 906 B1**
(45) Veröffentlichungstag und Bekanntmachung des Hinweises auf die Patenterteilung: **08.09.1993**
(21) Anmeldenummer: 88118389.1
(22) Anmeldetag: 04.11.1988
(51) Int. Cl.: C07C 205/37, C07C 201/12

(54) **Verfahren zur Herstellung von o-Nitrophenetol**
Process for the preparation of ortho-nitrophenetole
Procédé de préparation de l'ortho-nitrophénétole

(30) Priorität: 07.11.1987 DE 3737919
(43) Veröffentlichungstag der Anmeldung: 17.05.1989
(73) Patentinhaber: HOECHST AKTIENGESELLSCHAFT, 65926 Frankfurt am Main (DE)
(72) Erfinder: Heise, Hartmut, Dr., D-6232 Bad Soden am Taunus (DE); Hintzmann, Manfred, Dr., D-6238 Hofheim am Taunus (DE)

(56) Entgegenhaltungen:
- DE-A- 2 634 419
- DE-A- 3 120 912
- DE-A- 3 307 164

## Beschreibung

Gegenstand der vorliegenden Erfindung ist ein verbessertes Verfahren zur Herstellung von praktisch reinem o-Nitrophenetol durch Ethoxylierung von o-Nitrochlorbenzol.

o-Nitrophenetol ist Vorprodukt zur Herstellung von o-Phenetidin, welches seinerseits ein wichtiges Zwischenprodukt zur Herstellung von Farbstoffen und Pharmazeutika ist. Es hat daher in der Vergangenheit nicht an Versuchen gefehlt, o-Nitrophenetol in einfacher Weise und mit hohen Ausbeuten herstellen zu können.

In der Literatur sind verschiedene Verfahren zur Herstellung von o-Nitrophenetol durch Ethoxylierung von o-Nitrochlorbenzol beschrieben worden. Danach wird o-Nitrochlorbenzol mit Ethanol in Gegenwart von Alkalien umgesetzt (US-PS 2 545 597, GB-PS 902 306, C.A. 34 5423 (1940), US-PS 3 085 113 und J. scient. ind. Res. India 4 (1945) 369 bzw. 5 B (1946) 25).

Der wesentliche Nachteil dieser bekannten Verfahren ist das verstärkte Auftreten von Nebenreaktionen. Als Nebenprodukte bilden sich hauptsächlich Azoxyverbindungen sowie o-Chloranilin und o-Nitrophenol. Um die Nebenreaktionen ganz oder teilweise zu unterdrücken, sind in der Vergangenheit verschiedene Vorschläge gemacht worden. So werden in der US-PS 2 545 597 Nebenreaktionen verhindert, indem man die Ethoxylierung in Gegenwart von Mangannaphthenat und unter Einleiten von Luft in die Reaktionslösung durchführt.

Es ist auch versucht worden, die Bildung von Azoxybenzolen dadurch zu unterdrücken, daß man die Umsetzung von o-Nitrochlorbenzol mit ethanolischer Natronlauge in Gegenwart von Metalloxiden, wie PbO₂ oder MnO₂, durchführt, wie dies in J. scient. ind. Res. India 4 (1945) 369,372 und 5 B (1946) 25 vorgeschlagen wird. Dennoch konnten Nebenreaktionen, die beispielsweise zur Bildung von Azoxyverbindungen führen, nie ganz unterdrückt werden, so daß nach Aufarbeitung des Reaktionsproduktes sich zusätzlich Reinigungsschritte anschließen mußten. Ein weiterer Nachteil dieser Verfahren ist, daß die Ausbeuten an o-Nitrophenetol meist nicht über 90% liegen und daß ein annähernd vollständiger Umsatz nicht erreicht wird. Der nicht umgesetzte Anteil an o-Nitrochlorbenzol liegt im Bereich von 3 bis 6 % vom Einsatz, was weitere Reinigungsschritte bei der Aufarbeitung erfordert.

Um diese Nachteile auszuschalten, ist bereits versucht worden, die Ethoxylierung von Nitrochlorbenzolen in Gegenwart von Phasentransfer-Katalysatoren durchzuführen. Als für derartige Reaktionen geeignete Katalysatoren sind organische quartäre Ammoniumsalze verwendet worden. So wird beispielsweise in der DE-OS 2 634 419 die Umsetzung von p-Nitrochlorbenzol mit Ethanol in 50%iger Natronlauge und in Gegenwart von Cetyltrimethylammoniumbromid als Phasentransfer-Katalysator beschrieben. Einähnliches Verfahren zur Herstellung von p-Nitrophenetol findet sich in der DE-OS 3 120 912. Die dabei eingesetzten Katalysatormengen betragen zwischen 8,5 und 12,5 Gew.-%, bezogen auf eingesetztes p-Nitrochlorbenzol, und die Ausbeuten an p-Nitrophenetol liegen bei 90 bis 95 %, während der Rest im wesentlichen aus Dichlorazoxybenzol besteht.

Während sich die bisher beschriebenen bekannten Verfahren in Gegenwart von Phasentransfer-Katalysatoren ausschließlich auf die Herstellung von p-Nitrophenetol beziehen, ist in der DE-OS 3 307 164 neben der Herstellung des p-Isomeren erstmals auch die Ethoxylierung von o-Nitrochlorbenzol in konzentrierter Natronlauge und in Gegenwart eines Tetramethylammoniumsalzes als Phasentransfer-Katalysator beschrieben worden. Danach sollen bei diesem Verfahren p-Nitrophenetol und analog dazu o-Nitrophenetol "vollständig frei von irgendwelchen Nebenreaktionsprodukten" und in sehr hohen Ausbeuten von über 97 %, bezogen auf eingesetztes o-Nitrochlorbenzol, erhalten werden.
Da die Ethoxylierung von o-Nitrochlorbenzol erfahrungsgemäß sehr viel stärker zur Bildung von Nebenprodukten führt als dies beim p-Isomeren der Fall ist, muß es erstaunen, daß hier das Verfahren zur Herstellung von p-Nitrophenetol einfach auf das o-Isomere übertragen wird. Bei der Nacharbeitung des in Beispiel 3 der DE-OS 3 307 164 beschriebenen Verfahrens (dieses Beispiel ist das einzige, welches auf die Herstellung des o-Nitrophenetols gerichtet ist), ist es trotz mehrfacher Versuche und trotz genauer Einhaltung der dort angegebenen Verfahrensbedingungen in keinem Fall gelungen, ein Produkt zu erhalten, welches frei von Nebenprodukten ist. Vielmehr enthielt das nach dem genannten Beispiel hergestellte o-Nitrophenetol stets erhebliche Mengen an Nebenprodukten des Azoxy-Typs sowie o-Chloranilin, o-Nitrophenol und andere nicht weiter identifizierte Substanzen.

Es war daher die Aufgabe gestellt, ein Verfahren zur Herstellung von o-Nitrophenetol durch Ethoxylierung von o-Nitrochlorbenzol in konzentrierter Alkalilauge und in Gegenwart eines Phasentransfer-Katalysators zu entwickeln, das den in der DE-OS 3 307 164 geforderten Qualitätsmerkmalen, die nach dem dort beschriebenen Verfahren nicht erreicht werden, tatsächlich genügt.

Es wurde nun überraschenderweise gefunden, daß man o-Nitrophenetol vollkommen frei von Azoxyverbindungen und in hohen Ausbeuten herstellen kann, indem man auf 1 Mol o-Nitrochlorbenzol 1,05 bis 1,4 Mol, vorzugsweise 1,1 bis 1,2 Mol Ethanol in Gegenwart eines Phasentransfer-Katalysators in 40 bis 70 gewichtsprozentiger, vorzugsweise 50 bis 60%iger, Alkalimetallhydroxidlösung bei Temperaturen von 50 bis 80°C, vorzugsweise von 65 bis 70°C, derart einwirken läßt, daß die Ethanolkonzentration der Reaktionsmischung 1,5 Gewichtsprozent während des gesamten Reaktionsablaufs nicht übersteigt, vorzugsweise sich zwischen 0,2 und 0,8 Gewichtsprozent bewegt. Letzteres kann dadurch erreicht werden, daß man das Ethanol der Reaktionslösung entsprechend zudosiert.

Darüberhinaus wurde überraschenderweise gefunden, daß der Umsatz des o-Nitrochlorbenzols praktisch vollständig erreicht werden kann, indem man auch den Phasentransfer-Katalysator dem Reaktionsgemisch zudosiert. Dies kann unabhängig von oder zweckmäßigerweise zusammen mit der Ethanolzudosierung erfolgen.

Die Zugabe des Ethanols getrennt oder gemeinsam mit dem Phasentransfer-Katalysator kann in der Weise vorgenommen werden, daß man stets gleiche Anteile pro Zeiteinheit zudosiert, oder aber entsprechend dem Ablauf der Reaktion zunächst schneller und gegen Ende langsamer zudosiert. Besonders bewährt hat sich dabei die gemeinsame Zudosierung von Katalysator und Ethanol, und zwar in der Weise, daß man etwa 60 bis etwa 80 Gewichtsprozent der zur Anwendung gelangenden Menge schnell in einer bestimmten Zeiteinheit zudosiert und die Restmenge anschließend in der 2- bis 4-fachen Zeiteinheit. Auf diese Weise ist gewährleistet, daß eine bestimmte Ethanolkonzentration während des gesamten Reaktionsablaufs nicht überschritten wird. Wie lang die Dosierzeiten bei der Durchführung des erfindungsgemäßen Verfahrens tatsächlich sind, hängt beispielsweise von der Reaktionstemperatur und von der Rührung bzw. der guten Durchmischung des Reaktionsansatzes ab. Zeitbestimmender Faktor ist allein der Fortgang der Reaktion bzw. die im Reaktionsgemisch jeweils vorhandene Ethanolkonzentration, die in einem Pilotansatz ermittelt werden muß.

Das insgesamt zur Anwendung kommende Ethanol wird stets im Überschuß eingesetzt, und zwar in einem Verhältnis von etwa 1,05 bis etwa 1,4 Mol Ethanol pro Mol vorgelegtes o-Nitrochlorbenzol. Als besonders vorteilhaft hat sich dabei ein Molverhältnis von etwa 1,1 bis etwa 1,2 Mol Ethanol pro Mol o-Nitrochlorbenzol erwiesen.

Als Phasentransfer-Katalysatoren kommen beim erfindungsgemäßen Verfahren insbesondere quartäre Ammoniumsalze der allgemeinen Formel 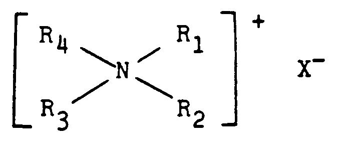
in welcher R₁, R₂, R₃ und R₄ gleiche oder verschiedene Kohlenwasserstoffreste mit einer Gesamtzahl von etwa 10 bis etwa 50 Kohlenstoffatomen, und X⁻ ein Halogenidion, vorzugsweise ein Chlorid- oder Bromidion, ferner ein Hydrogensulfation (HSO₄⁻) oder Hydroxylion bedeuten, in Betracht. An bewährten Ammoniumsalzen seien im einzelnen folgende genannt:
Alkylbenzyldimethylammoniumchloride, Benzyltributylammoniumchlorid oder -bromid, Benzyltriäthylammoniumchlorid, Benzyltriäthylammoniumhydroxid, Benzyltrimethylammoniumchlorid, Benzyltrimethylammoniumhydroxid, Äthylhexadecyldimethylammoniumbromid, Hexadecyltrimethylammoniumchlorid oder -bromid, Methyltrioctylammoniumchlorid, Tetrabutylammoniumhydrogensulfat, Tetrabutylammoniumhydroxid, Tetrabutylammoniumbromid oder -jodid, Tetraäthylammoniumchlorid oder -bromid, Tetraäthylammoniumhydroxid, Tetraoctylammoniumbromid, Tetrapropylammoniumbromid und Tributylmethylammoniumchlorid.

Besonders bewährt hat sich Dimethylbenzylcocosalkyl-(C₁₀-C₁₈)-ammoniumchlorid mit einem durchschnittlichen Molgewicht von 382,5.

Der Katalysator kann sowohl allein als auch im Gemisch mit anderen Phasentransfer-Katalysatoren verwendet werden. Die Katalysatoren der genannten Art können in Form wäßriger oder ethanolischer Lösungen eingesetzt werden.

Die genannten Katalysatoren können in einer Menge von etwa 3 bis etwa 15 Gewichtsprozent, bezogen auf vorgelegtes o-Nitrochlorbenzol, zur Anwendung gelangen.

Das verwendete Alkalimetallhydroxid ist vorzugsweise Natrium- oder Kaliumhydroxid. Auch Gemische davon sind geeignet. Die angewandten Konzentrationen können in einem weiten Bereich zwischen etwa 40 und etwa 70 Gewichtsprozent schwanken, wobei 50 bis 60%ige wäßrige Lösungen bevorzugt sind.

Bei der Verwendung wäßriger Katalysatorlßösungen ist das in die Reaktion eingebrachte Wasser bei der Ermittlung der Alkalimetallhydroxid-Konzentration entsprechend zu berücksichtigen und gegebenenfalls durch Zusatz von festem Natrium- oder Kaliumhydroxid auszugleichen. Das Alkalimetallhydroxid wird zweckmäßigerweise in einem etwa 2-bis etwa 6-fachen molaren Überschuß, bezogen auf vorgelegtes o-Nitrochlorbenzol, eingesetzt, wobei in der bevorzugten Ausführungsform ein etwa 4- bis 5-facher molarer Überschuß angewandt wird.

Die Durchführung des erfindungsgemäßen Verfahrens erfolgt zweckmäßigerweise derart, daß o-Nitrochlorbenzol zusammen mit dem wäßrigen Alkalimetallhydroxid vorgelegt und auf etwa 65 bis etwa 68°C erwärmt wird. Unter kräftigem Rühren und vorsichtigem Kühlen beginnt man mit der Dosierung der Lösung aus Katalysator und Ethanol, und zwar mit einer derartigen Dosiergeschwindigkeit, daß eine Ethanol-Konzentration im Reaktionsgemisch von etwa 0,5 bis etwa 0,6 % nicht überschritten wird. Nachdem 3/4 der benötigten Ethanol- und Katalysatormengen in einer vom Ethanolgehalt im Reaktionsgemisch abhängigen Zeiteinheit dosiert sind, wird die Dosiergeschwindigkeit herabgesetzt, so daß die restliche Menge in der doppelten Zeiteinheit einläuft. Dabei soll die Reaktionstemperatur von 70°C nicht überschritten werden. Nach beendeter Dosierung rührt man nach, bis die Konzentration an noch nicht umgesetztem o-Nitrochlorbenzol unter 1 % gesunken ist, was im allgemeinen nach einer Gesamtreaktionszeit von 8 bis 9 Stunden der Fall ist.

Die nachstehenden Beispiele dienen zur Erläuterung des erfindungsgemäßen Verfahrens, ohne es darauf zu beschränken.

### Beispiel 1

Zu einem Gemisch aus 788 g o-Nitrochlorbenzol, 1 600 g 50%iger Natronlauge und 260 g Natriumhydroxid pumpt man unter kräftigem Rühren bei 68 bis 70°C eine Lösung von 253,5 g Ethanol und 120 g Dimethylbenzylcocosalkyl(C₁₀-C₁₈)-ammoniumchlorid (50%ige wäßrige Lösung), und zwar so, daß während 60 Minuten 280 g und während der folgenden 120 Minuten 93,5 g der ethanolischen Katalysator-Lösung in das Reaktionsgemisch gelangen.
Um die Reaktionstemperatur von 68 bis 70°C aufrecht zu erhalten wird zu Beginn der Umsetzung gekühlt, später geheizt. Nach beendetem Zulauf der Ethanol/Katalysatorlösung wird noch 6 Stunden bei 68 bis 70°C weitergerührt. Der gaschromatographisch bestimmte Gehalt an o-Nitrochlorbenzol beträgt danach < 1 %.
Nach Phasentrennung werden 917,5 g eines feuchten, katalysatorhaltigen Produkts mit einem Reingehalt von 85,8 % erhalten, was einer Ausbeute von 94,2 % entspricht. Das Produkt ist vollkommen frei von Azoxyverbindungen.

### Beispiel 2

In ein Gemisch aus 392,5 g o-Nitrochlorbenzol, 800 g 50%iger Natronlauge und 100 g Natriumhydroxid pumpt man unter kräftigem Rühren bei 68 bis 70°C eine Lösung von 30 g Tetrabutylammoniumbromid in 126,8 g Ethanol so ein, daß 120 g während der ersten 60 Minuten und 36,8 g der Lösung während der folgenden 120 Minuten einlaufen. Nach beendetem Einlauf und nach 6-stündiger Nachrührzeit bei 70°C beträgt der gaschromatographisch bestimmte Gehalt an o-Nitrochlorbenzol 0,6 %.
Nach Phasentrennung werden 451 g feuchtes Produkt mit einem Reingehalt von 88,6 % erhalten, entsprechend einer Ausbeute von 95,6 %.

### Beispiel 3

In ein auf 68 bis 70°C erwärmtes und gerührtes Gemisch aus 78,8 g o-Nitrochlorbenzol, 160 g Natronlauge (50%ig) und 6 g Tetrabutylammoniumbromid dosiert man innerhalb von 1 Stunde 20 g Ethanol. Die restlichen 5,4 g Ethanol werden anschließend während 2 Stunden dosiert. Am Ende der Nachrührzeit von 6 Stunden bei 70°C war ein Umsatz von 97,4 % erreicht. Ein Umsatz von 99,2 % wurde nach 16stündigem Nachrühren (70°C) erreicht.

## Patentansprüche

1. Verfahren zur Herstellung von o-Nitrophenetol, dadurch gekennzeichnet, daß man auf 1 Mol o-Nitrochlorbenzol 1,05 bis 1,4 Mol Ethanol in Gegenwart eines Phasentransfer-Katalysators in 40 bis 70 gewichtsprozentiger Alkalimetallhydroxidlösung bei Temperaturen von 50 bis 80°C derart einwirken läßt, daß die Ethanolkonzentration der Reaktionsmischung während des gesamten Reaktionsablaufs 1,5 Gewichtsprozent nicht übersteigt.

2. Verfahren nach Anspruch 1, dadurch gekennzeichnet, daß sich die Ethanolkonzentration der Reaktionsmischung zwischen 0,2 und 0,8 Gewichtsprozent bewegt.

3. Verfahren nach mindestens einem der Ansprüche 1 und 2, dadurch gekennzeichnet, daß man das Ethanol dem zusammen mit der wäßrigem Alkalimetallhydroxidlösung vorgelegten o-Nitrochlorbenzol in Abhängigkeit vom Fortgang der Reaktion zudosiert.

4. Verfahren nach mindestens einem der Ansprüche 1 bis 3, dadurch gekennnzeichnet, daß man bei Temperaturen von 65 bis 70°C umsetzt.

5. Verfahren nach mindestens einem der Ansprüche 1 bis 4, dadurch gekennzeichnet, daß man als wäßrige Alkalimetallhydroxidlösung eine wäßrige Natriumhydroxidlösung oder Kaliumhydroxidlösung oder Mischungen daraus verwendet.

6. Verfahren nach mindestens einem der Ansprüche 1 bis 5, dadurch gekennzeichnet, daß man das Ethanol und den Phasentransfer-Katalysator gleichzeitig aber getrennt dem zusammen mit der wäßrigen Alkalimetallhydroxidlösung vorgelegten o-Nitrochlorbenzol zudosiert.

7. Verfahren nach mindestens einem der Ansprüche 1 bis 6, dadurch gekennzeichnet, daß man das Ethanol und den Phasentransfer-Katalysator gleichzeitig und getrennt oder zusammen dem mit der wäßrigen Alkalimetallhydroxidlösung vorgelegten o-Nitrochlorbenzol derart zudosiert, daß beide Komponenten in gleichen oder verschiedenen Anteilen pro Zeiteinheit zudosiert werden.

8. Verfahren nach mindestens einem der Ansprüche 1 bis 5 und 7, dadurch gekennzeichnet, daß man das Ethanol und den Phasentransfer Katalysator gleichzeitig und zusammen dem mit der wäßrigen Alkalimetallhydroxidlösung vorgelegten o-Nitrochlorbenzol derart zudosiert, daß man 60 bis 80 Gew.-% des insgesamt eingesetzten Ethanols schnell in einer bestimmten Zeiteinheit zudosiert und anschließend die Restmenge des Ethanols in der 2- bis 4-fachen Zeiteinheit zudosiert.

9. Verfahren nach mindestens einem der Ansprüche 1 bis 8, dadurch gekennzeichnet, daß man als Phasentransfer-Katalysatoren quartäre Ammoniumsalze der allgemeinen Formel 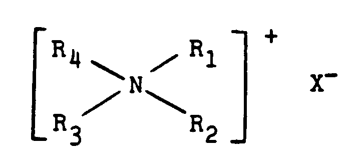 in welcher R₁, R₂, R₃ und R₄ gleiche oder verschiedene Kohlenwasserstoffreste mit einer Gesamtzahl von etwa 10 bis etwa 50 Kohlenstoffatomen und X⁻ ein Halogenidion, Hydrogensulfation (HSO₄⁻) oder Hydroxylion bedeuten, verwendet.

10. Verfahren nach mindestens einem der Ansprüche 1 bis 9, dadurch gekennzeichnet, daß man als Phasentransfer-Katalysator Dimethylbenzylcocosalkyl(C₁₀-C₁₈)-ammoniumchlorid, Benzyltributylammoniumchlorid oder -bromid, Benzyltriäthylammoniumchlorid, Benzyltriäthylammoniumhydroxid, Benzyltrimethylammoniumchlorid, Benzyltrimethylammoniumhydroxid, Äthylhexadecyldimethylammoniumbromid, Hexadecyltrimethylammoniumchlorid oder -bromid, Methyltrioctylammoniumchlorid, Tetrabutylammoniumhydrogensulfat, Tetrabutylammoniumhydroxid, Tetrabutylammoniumbromid oder -jodid, Tetraäthylammoniumchlorid oder -bromid, Tetraäthylammoniumhydroxid, Tetraoctylammoniumbromid, Tetrapropylammoniumbromid oder Tributylmethylammoniumchlorid verwendet.

## Claims

1. A process for the preparation of o-nitrophenetole, which comprises allowing 1.05 to 1.4 mole of ethanol to act on 1 mole of o-nitrochlorobenzene in the presence of a phase-transfer catalyst in 40 to 70 per cent by weight alkali metal hydroxide solution at temperatures from 50 to 80°C in such a way that the ethanol concentration in the reaction mixture does not exceed 1.5 per cent by weight throughout the course of the reaction.

2. The process as claimed in claim 1, wherein the ethanol concentration in the reaction mixture ranges between 0.2 and 0.8 per cent by weight.

3. The process as claimed in at least one of claims 1 and 2, wherein the metering of the ethanol into the o-nitrochlorobenzene which has been introduced together with the aqueous alkali metal hydroxide solution takes place as a function of the progress of the reaction.

4. The process as claimed in at least one of claims 1 to 3, wherein the reaction is carried out at temperatures from 65 to 70°C.

5. The process as claimed in at least one of claims 1 to 4, wherein an aqueous sodium hydroxide solution or potassium hydroxide solution, or mixtures thereof, is used as aqueous alkali metal hydroxide solution.

6. The process as claimed in at least one of claims 1 to 5, wherein the ethanol and the phase-transfer catalyst are metered simultaneously but separately into the o-nitrochlorobenzene, which has been introduced together with the aqueous alkali metal hydroxide solution.

7. The process as claimed in at least one of claims 1 to 6, wherein the ethanol and the phase-transfer catalyst are metered simultaneously and separately or together into the o-nitrochlorobenzene, which has been introduced with the aqueous alkali metal hydroxide solution, in such a way that equal or different portions of the two components are metered in per unit time.

8. The process as claimed in at least one of claims 1 to 5 and 7, wherein the ethanol and the phase-transfer catalyst are metered simultaneously and together into the o-nitrochlorobenzene, which has been introduced with the aqueous alkali metal hydroxide solution, in such a way that 60 to 80 % by weight of the total amount of ethanol used is rapidly metered in during a particular unit time, and subsequently the remaining amount of ethanol is metered in during 2 to 4 times the unit time.

9. The process as claimed in at least one of claims 1 to 8, wherein quaternary ammonium salts of the general formula 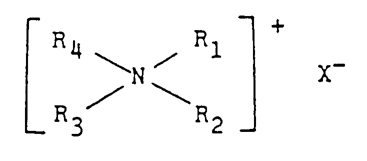 in which R₁, R₂, R₃ and R₄ denote identical or different hydrocarbon radicals having a total number of about 10 to 50 carbon atoms, and X⁻ denotes a halide ion, bisulfate ion (HSO₄⁻) or hydroxyl ion, are used as phase-transfer catalysts.

10. The process as claimed in at least one of claims 1 to 9, wherein dimethylbenzyl-coconut alkyl(C₁₀-C₁₈)-ammonium chloride, benzyltributylammonium chloride or bromide, benzyltriethylammonium chloride, benzyltriethylammonium hydroxide, benzyltrimethylammonium chloride, benzyltrimethylammonium hydroxide, ethylhexadecyldimethylammonium bromide, hexadecyltrimethylammonium chloride or bromide, methyltrioctylammonium chloride, tetrabutylammonium bisulfate, tetrabutylammonium hydroxide, tetrabutylammonium bromide or iodide, tetraethylammonium chloride or bromide, tetraethylammonium hydroxide, tetraoctylammonium bromide, tetrapropylammonium bromide or tributylmethylammonium chloride is used as phase-transfer catalyst.

## Revendications

1. Procédé pour préparer l'o-nitrophénétole, caractérisé en ce qu'on fait agir sur une mole d'o-nitrochlorobenzène 1,05 à 1,4 mole d'éthanol en présence d'un catalyseur de transfert de phases dans une solution contenant 40 à 70 % en poids d'un hydroxyde de métal alcalin, à des températures de 50 à 80°C, de manière que la concentration en éthanol du mélange réactionnel n'excède pas 1,5 % en poids pendant la totalité du déroulement de la réaction.

2. Procédé selon la revendication 1, caractérisé en ce que la concentration en éthanol du mélange réactionnel varie entre 0,2 et 0,8 % en poids.

3. Procédé selon l'une au moins des revendications 1 et 2, caractérisé en ce qu'on ajoute de façon dosée, en fonction du déroulement de la réaction, l'éthanol à l'o-nitrochlorobenzène placé au préalable avec la solution aqueuse d'hydroxyde de métal alcalin.

4. Procédé selon l'une au moins des revendications 1 à 3, caractérisé en ce qu'on effectue la réaction à des températures de 65 à 70°C.

5. Procédé selon l'une au moins des revendications 1 à 4, caractérisé en ce qu'on utilise comme solution aqueuse d'hydroxyde de métal alcalin une solution aqueuse d'hydroxyde de sodium ou une solution aqueuse d'hydroxyde de potassium ou des mélanges de ces solutions.

6. Procédé selon l'une au moins des revendications 1 à 5, caractérisé en ce qu'on ajoute de façon dosée l'éthanol et le catalyseur de transfert des phases, simultanément mais de manière séparée à l'o-nitrochlorobenzène placé au préalable avec la solution aqueuse d'hydroxyde de métal alcalin.

7. Procédé selon l'une au moins des revendications 1 à 6, caractérisé en ce qu'on ajoute de façon dosée l'éthanol et le catalyseur de transfert de phases, simultanément et séparémment ou bien ensemble à l'o-nitrochlorobenzène placé au préalable avec la solution aqueuse d'hydroxyde de métal alcalin, l'addition étant effectuée de manière que les deux constituants sont ajoutés en des proportions égales ou différentes par une unité d'intervalle de temps.

8. Procédé selon l'une au moins des revendications 1 à 5 et 7, caractérisé en ce qu'on ajoute de façon dosée l'éthanol et le catalyseur de transfert des phases, simultanément et ensemble à l'o-nitrochlorobenzène, placé au préalable avec la solution aqueuse à l'hydroxyde de métal alcalin, l'addition étant réalisée de manière que 60 ou 80 % en poids de l'éthanol utilisé total soient introduits de façon dosée rapidement en un intervalle déterminé de temps et qu'ensuite la quantité restante de l'éthanol soit ajoutée en un intervalle de temps double à quadruple.

9. Procédé selon l'une au moins des revendications 1 à 8, caractérisé en ce qu'on utilise comme catalyseurs de transfert de phases des sels d'ammonium quaternaire de formule générale : 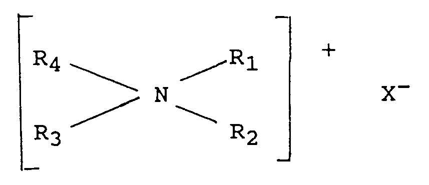 dans laquelle R₁, R₂, R₃ et R₄ représentent des restes d'hydrocarbures identiques ou différents ayant un nombre total d'atomes de carbone d'environ 10 à environ 50, et X⁻ est un ion halogénure, un ion hydrogénosulfate (HSO₄⁻ ou un ion hydroxyle.

10. Procédé selon l'une au moins des revendications 1 à 9, caractérisé en ce qu'on utilise,comme catalyseur de transfert de phases, du chlorure de diméthylbenzylalkyl (en C₁₀-C₁₈, de noix de coco) ammonium, le chlorure ou le bromure de benzyltributylammonium, le chlorure de benzyltriéthylammonium, l'hydroxyde de benzyltriéthylammonium, le chlorure de benzyltriméthylammonium, l'hydroxyde de benzyltriméthylammonium, le bromure d'éthylhexadécyldiméthylammonium, le chlorure ou le bromure d'hexadécyltriméthylammonium, le chlorure de méthyltrioctylammonium, l'hydrogénosulfate de tétrabutylammonium, l'hydroxyde de tétrabutylammonium, le bromure ou l'iodure de tétrabutylammonium, le chlorure ou le bromure de tétraéthylammonium, l'hydroxyde de tétraéthylammonium, le bromure de tétraoctylammonium, le bromure de tétrapropylammonium ou le chlorure de tributylméthylammonium.
